# EUROPÄISCHE PATENTANMELDUNG

(11) **EP 2 194 278 A1**
(43) Veröffentlichungstag der Anmeldung: **09.06.2010**
(21) Anmeldenummer: 08075923.6
(22) Anmeldetag: 05.12.2008
(51) Int. Cl.: F04D 29/02, F04D 29/24, A61M 1/10

(54) **Fluidpumpe mit einem rotor**

(71) Anmelder: ECP Entwicklungsgesellschaft mbH, 12247 Berlin (DE)
(72) Erfinder: Scheckel, Mario, 13187 Berlin (DE)
(74) Vertreter: Pfenning, Meinig & Partner GbR

(57) **Zusammenfassung**

Die Erfindung bezieht sich auf eine Fluidpumpe, insbesondere auf eine Flüssigkeitspumpe mit einem Rotor (18) mit wenigstens einem Rotorblatt (20, 21) zur Förderung des Fluids, wobei der Rotor bezüglich seines Durchmessers zwischen einem ersten, komprimierten Zustand und einem zweiten, expandierten Zustand veränderbar ist. Zur Realisierung einer einfachen Komprimierbarkeit und Expandierbarkeit des Rotors der Pumpe ist gemäß der Erfindung vorgesehen, dass das wenigstens eine Rotorblatt zwischen einem ersten Zustand, den es im komprimierten Zustand des Rotors einnimmt, und einem zweiten Zustand, den es im expandierten Zustand des Rotors einnimmt, durch einen Fluidgegendruck (23) bei einer Rotation des Rotors im Pumpbetrieb verformbar ist.

## Beschreibung

Die Erfindung liegt auf dem Gebiet der Fluidpumpen und betrifft eine Pumpe, die bezüglich ihres Rotordurchmessers veränderbar ist, um beispielsweise durch enge Öffnungen, wie Röhren, insbesondere Blutgefäße, durchgeführt und nach der Durchführung in expandiertem Zustand betrieben werden zu können.

Die Anwendung kann somit einerseits im medizinischen Bereich, beispielsweise als Blutpumpe zur Herzunterstützung, in minimalinvasiver Weise vorgesehen sein, andererseits ist jedoch auch ein Einsatz in Rührwerken oder als Vortriebselement für Schiffe denkbar.

Besondere Vorteile kann die Erfindung durch die mögliche Miniaturisierung im medizinischen Bereich entfalten.

Dort kann, nach Einführung der Fluidpumpe durch ein großes Blutgefäß bis in die Herzkammer und nachfolgende Inbetriebnahme nach Expansion des Rotors, die Pumpleistung eines Herzens beispielsweise beim Menschen erheblich unterstützt oder teilweise ersetzt werden. Der therapeutische Vorteil solcher Anwendungen liegt in einer zumindest teilweisen Entlastung des Herzmuskels.

Derartige expandierbare Fluidpumpen sind aus dem Stand der Technik bereits bekannt. Beispielsweise geht aus der DE 10 059 714 C1 eine Pumpe hervor, die mitsamt dem Pumpenantrieb durch ein Blutgefäß geschoben werden kann. Das Blut fließt dort durch eine Kanüle, deren Durchmesser zur Veränderung der Strömungsverhältnisse expandierbar und komprimierbar ist.

Aus der WO 03/103745 A2 ist eine Blutpumpe bekannt, deren Rotor radial komprimierbar und expandierbar ist, wobei dort verschiedene Konstruktionen zur Erreichung der Expandierbarkeit vorgeschlagen werden. Beispielsweise kann durch verschiedene gegeneinander verschiebbare Teile des Katheters nach der Einführung eine Stauchung des Pumpengehäuses und damit verbunden eine radiale Aufweitung erfolgen. Andererseits ist die Möglichkeit offenbart, durch Drehung einer Antriebswelle gegenüber einem in dem Katheter befindlichen Draht eine Helixstruktur des Drahtes zu erzeugen, wobei der Draht zudem eine Membran trägt, die nach Annahme der Helixstruktur eine Rotorschaufel bildet.

Zudem ist aus dem Dokument eine Rotorstruktur mit einer Mehrzahl von in sich steifen, schwenkbar an einem zentralen Teil angelenkten Schaufeln bekannt, die sich im Betrieb aufstellen und damit einen Fluiddruck erzeugen.

Aus der EP 0 768 900 B1 ist eine Pumpe bekannt, bei der innerhalb eines Pumpengehäuses an eine Welle Rotorblätter derart angelenkt sind, dass sie im Ruhezustand an die Welle angeklappt und im Betrieb senkrecht zur Welle aufgestellt werden können, um das Fluid zu fördern.

Dem bekannten Stand der Technik ist gemein, dass Rotorblätter einer Pumpe zur Expansion entweder mittels eines Schwenkmechanismus aufgeschwenkt werden oder durch eine mechanische Vorrichtung nach Art eines Bowdenzuges oder ähnlich erst zur Expansion der Pumpe gebildet werden.

Der vorliegenden Erfindung liegt vor dem Hintergrund des Standes der Technik die Aufgabe zugrunde, eine Fluidpumpe mit einem bezüglich seines Durchmessers komprimierbaren Rotor zu schaffen, der konstruktiv möglichst einfach aufgebaut ist, der vorzugsweise wie das ihn umgebende Pumpengehäuse aus biokompatiblen Materialien besteht, dessen Expansion und Kompression möglichst einfach bewerkstelligt werden können und der im Betrieb die notwendige Zuverlässigkeit aufweist.

Die Aufgabe wird gemäß der Erfindung mit den Merkmalen des Patentanspruchs 1 gelöst.

Zudem bezieht sich die Erfindung auf Verfahren zum Betrieb der erfindungsgemäßen Fluidpumpe gemäß den Ansprüchen 16, 17 oder 18.

Der Erfindung liegt die Erkenntnis zugrunde, dass eine möglichst einfache Struktur der Fluidpumpe durch eine Verformbarkeit eines Rotorblattes selbst zu realisieren ist. Der Rotor der Fluidpumpe weist dazu wenigstens ein Rotorblatt auf, das sich in einem ersten Zustand befindet, so lange der Rotor einen ersten, komprimierten Zustand einnimmt, wobei das Rotorblatt bei Übergang des Rotors in einem expandierten Zustand durch Verformung einen zweiten Zustand einnimmt.

Dabei wird das Rotorblatt von dem ersten Zustand in den zweiten Zustand durch den Fluidgegendruck überführt, der bei der Rotation des Rotors im Pumpbetrieb auftritt.

Ein besonderer Vorteil der Erfindung liegt darin, dass keine Betätigungselemente zur Expansion des Rotors außer dem eigentlichen Antrieb der Pumpe vorgesehen werden müssen und dass durch die Verformbarkeit des/der Rotorblätter in sich auch keine schwenkbare Anlenkung von Rotorblättern an anderen Teilen der Pumpe vorgesehen werden muss.

Es tritt auch bei der Durchführung der Fluidpumpe durch eine Röhre, beispielsweise ein Blutgefäß, kein Bestreben des Rotors auf, ohne äußere Einflüsse zu expandieren. Ein derartiges Bestreben wäre bei medizinischem Einsatz nicht wünschenswert, da die Wände der Blutgefäße durch die die Pumpe durchgeführt wird, geschont werden sollen. Bei der Applikation durch einen röhrenförmigen künstlichen Zugang (Schleuse) würden die beschriebenen Rückstellkräfte eine besondere Schwierigkeit darstellen, da hierdurch hohe Reibungskräfte an der Wand der künstlichen Röhre entstünden und erhebliche Kräfte zum Vorschieben der Fluidpumpe in das Körperinnere erzeugt werden müssten.

Solange die Pumpe nicht betrieben, d.h. an der Pumpwelle nicht gedreht wird, bleibt der Rotor in dem komprimierten Zustand und kann durch das Blutgefäß vorgeschoben werden.

Wird die Pumpe an Ort und Stelle in Betrieb genommen, so wird der Rotor in Förderrichtung angetrieben und das/die Rotorblätter werden durch den Fluidgegendruck verformt und somit aufgestellt, wodurch die eigentliche, vollumfängliche Förderung in Gang kommt. Dabei ist es vorteilhaft, wenn die Verformung des/der Rotorblätter elastisch ist, da in vielen Anwendungsfällen die Fluidpumpe nach der Anwendung wieder komprimiert werden muss, um entfernt zu werden.

In diesem Fall nimmt das Rotorblatt/nehmen die Rotorblätter nach Einstellen des Pumpbetriebs und Stillsetzung des Rotors wieder ihren ersten Zustand ein, in dem der Rotor komprimiert ist.

Vorteilhaft ist ebenfalls, wenn die Verformbarkeit des wenigstens einen Rotorblatts durch Materialeigenschaften und/oder im konstruktiven Aufbau des Rotorblatts begrenzt ist.

Dabei soll die Grenze vorteilhaft dort liegen, wo durch die Verformung eine Form des Rotors angenommen wird, die die optimale Förderleistung erlaubt.

Dies kann gemäß der Erfindung vorteilhaft dadurch erreicht werden, dass das wenigstens eine Rotorblatt eine bei Förderbetrieb in Bewegungsrichtung vorlaufende und eine nachlaufende Seite aufweist, die verschieden gestaltet sind.

Üblicherweise wird die im Betrieb vorlaufende Seite des Rotorblattes vorwiegend auf Zug beansprucht, während die nachlaufende Seite einer Druckbeanspruchung unterliegt. Dabei kann die Trennfläche zwischen der vorlaufenden und der nachlaufenden Seite dort gedacht werden, wo eine neutrale Beanspruchung im Pumpbetrieb vorliegt. Diese Trennfläche muss entsprechende Schub- und Scherspannungen aufnehmen.

Es kann beispielsweise vorgesehen sein, dass die vorlaufende Seite und die nachlaufende Seite des Rotorblattes im Bereich der Trennfläche miteinander verklebt oder durch andere Fügetechniken miteinander verbunden sind.

Die für die Erfindung vorteilhaften Eigenschaften des Rotorblattes können beispielsweise dadurch erreicht werden, dass die vorlaufende Seite des wenigstens einen Rotorblatts aus einem ersten Material und die nachlaufende Seite aus einem zweiten, von dem ersten verschiedenen Material besteht.

Als vorteilhaft erweist es sich, wenn das erste Material dehnbarer ist als das zweite Material.

Das erste Material sollte dabei eine Dehngrenze aufweisen, so dass bei der Verformung des Rotorblattes im Pumpbetrieb eine möglichst genau definierte Grenze erreicht wird und sich eine definierte Form des Rotorblattes im Betrieb einstellt. Eine solche Dehngrenze ist beispielsweise durch einen nicht linearen Bereich der Elastizitätskoeffizienten des Materials gegeben, so dass die zur Dehnung notwendige Kraft ab einer bestimmten Dehngrenze überproportional ansteigt und die Form sich hierdurch stabilisiert. Diese Eigenschaft kann dem ersten Material eigen sein, sie kann jedoch dadurch unterstützt oder im Wesentlichen realisiert sein, dass in das erste Material dehnfeste Fasern eingebettet sind, die wesentlich dehnfester sind als das erste Material selbst und die im ersten Zustand des Rotorblattes ungestreckt, im zweiten Zustand in gestreckter Form in dem ersten Material vorliegen. Solche Fasern können beispielsweise durch hochfeste Kunststoffe oder durch Glas oder durch Kohlenstofffasern gebildet sein.

Das zweite Material auf der nachlaufenden Seite der Rotorblätter kann inkompressibel oder nur bis zu einer bestimmten Kompressibilitätsgrenze verformbar sein. Vorteilhaft ist die Verformbarkeit elastisch. Die Kompressionsgrenze kann beispielsweise durch eine Nichtlinearität der Kompressionskoeffizienten gebildet sein, indem die zur Kompression notwendige Kraft von einem bestimmten Kompressionsgrad an überproportional ansteigt.

Es kann auch vorteilhaft vorgesehen sein, dass das wenigstens eine Rotorblatt auf der nachlaufenden Seite Formelemente aufweist, die im ersten Zustand voneinander beabstandet sind und die im zweiten Zustand aneinander anliegen.

Diese Formelemente können im ersten Zustand durch Schlitze voneinander getrennt oder auch in ein komprimierbares Material eingebettet sein. Jedenfalls begrenzen sie, indem sie in dem zweiten Zustand aneinander anliegen, eine weitere Verformbarkeit des Rotorblattes.

Eine weitere vorteilhafte Ausgestaltung der Erfindung sieht vor, dass wenigstens ein Anschlagselement auf einer Seite des wenigstens einen Rotorblatts befestigt ist, welches die Trennfläche zwischen der vorlaufenden Seite und der nachlaufenden Seite durchsetzt und auf der anderen Seite des Rotorblatts in einer Ausnehmung begrenzt beweglich ist.

Das Anschlagselement ist vorteilhaft aus einem Material hergestellt, das annähernd so inkompressibel oder genauso inkompressibel ist wie das Material aus dem die nachlaufende Seite des Rotorblattes besteht, um eine definierte Anschlagsposition zu erzielen. Das Anschlagselement kann beispielsweise aus einem Metall oder einem harten Kunststoff bestehen.

Die Erfindung bezieht sich außer auf eine Fluidpumpe zudem auf ein Verfahren zum Betrieb einer Fluidpumpe der beschriebenen Form, wobei die Pumpe durch Rotation des Rotors in Betriebsrichtung gestartet und der Rotor durch den Fluidgegendruck expandiert wird.

Es kann zudem auch vorgesehen sein, dass zur Verringerung des Rotordurchmessers der Rotor in der der Betriebsrichtung entgegengesetzten Richtung angetrieben wird.

Es ist damit durch die Erfindung ermöglicht, dass der Rotor während des Durchführens der Pumpe durch eine Öffnung, insbesondere ein Blutgefäß in der der Betriebsrichtung entgegengesetzten Richtung angetrieben und damit komprimiert wird.

Die Erfindung wird nachfolgend anhand eines Ausführungsbeispiels in einer Zeichnung gezeigt und anschließend erläutert.

Dabei zeigt
- Fig. 1: schematisch die Anwendung einer Fluidpumpe in einem Herz zur Förderung von Blut,
- Fig. 2: schematisch einen Pumpenkopf im Längsschnitt mit radialer Anströmung,
- Fig. 2a: schematisch einen Pumpenkopf im Längsschnitt mit axialer Anströmung,
- Fig. 3: schematisch einen Rotor mit zwei Rotorblättern in einer Draufsicht,
- Fig. 4: einen Rotor in einer seitlichen Ansicht,
- Fig. 5: einen Schnitt durch einen Teil eines Rotorblattes,
- Fig. 6: einen Schnitt durch einen Teil eines Rotorblattes in einer anderen Ausgestaltung,
- Fig. 7: einen Schnitt durch einen Teil eines Rotorblattes,
- Fig. 8: eine Ausschnittvergrößerung des in der Fig. 7 mit VIII bezeichneten Details,
- Fig. 9: einen Schnitt durch ein Rotorblatt in einer weiteren Ausführungsform,
- Fig. 10: eine Ausführungsform eines Rotors mit einem helixförmigen Rotorblatt, das durch Formelemente gestützt ist,
- Fig. 11: einen Rotor, dessen helixförmiges Blatt durch eine schraubenförmige Wicklung gestützt ist, und
- Fig. 12: einen Rotor, dessen helixförmiges Rotorblatt durch eine Kulissenführung gestützt ist.

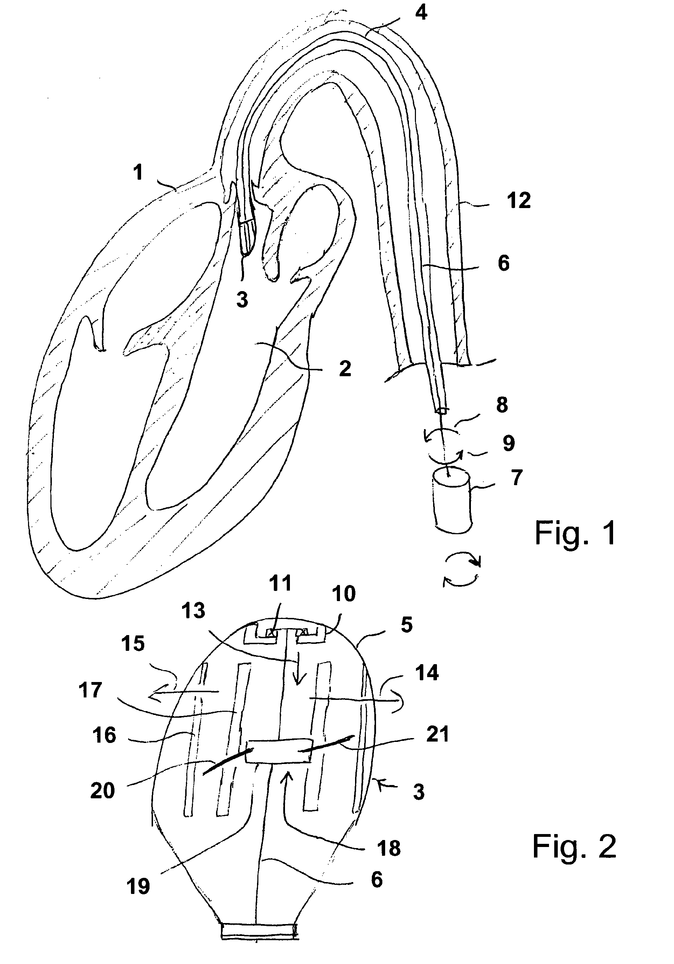

Die Fig. 1 zeigt schematisch im Querschnitt ein Herz 1, bei dem in eine Herzkammer 2 der Kopf 3 einer Fluidpumpe hineinragt. Der Pumpenkopf 3 ist am Ende einer Kanüle 4 angeordnet und weist ein vorn abgerundetes Pumpengehäuse 5 auf.

Der Antrieb der Pumpe erfolgt über eine Antriebswelle 6, die längs durch die Kanüle 4 verläuft und außen mit einem Motor 7 verbunden ist.

Der Motor 7 kann in beiden Richtungen 8, 9 angetrieben werden, wobei nur in einer Drehrichtung tatsächlich eine Förderung von Fluid stattfindet.

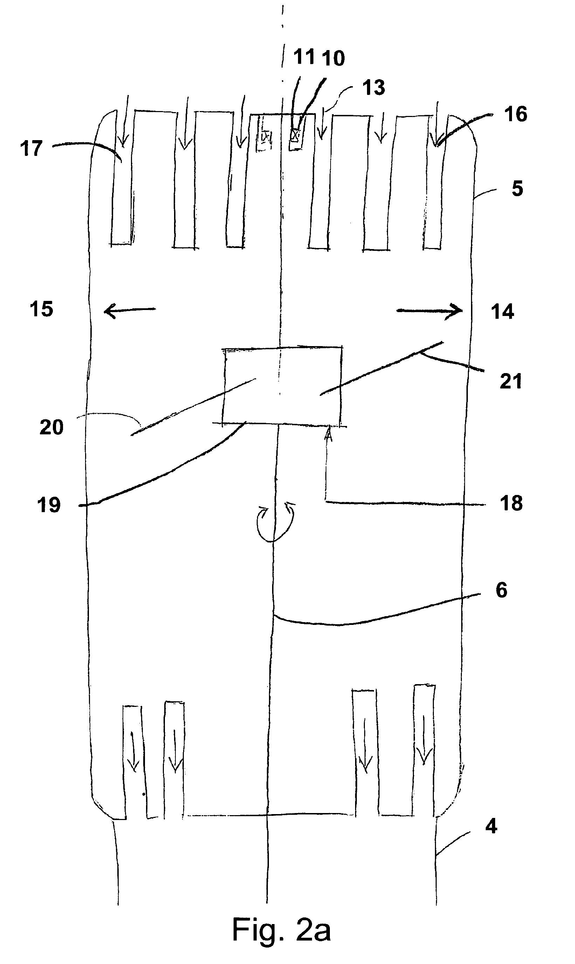

In der Fig. 2 ist schematisch im Längsschnitt der Pumpenkopf 3 mit dem Pumpengehäuse 5 gezeigt sowie die Antriebswelle 6. Diese ist am vorderen Ende des Pumpenkopfes 3 in einem Lagerbock 10 mittels eines Lagers 11 drehbar gelagert.

Die Fig. 2 zeigt den Pumpenkopf in einer expandierten Form, d.h. mit vergrößertem Radius gegenüber der Darstellung der Fig. 1.

Zur Einführung des Pumpenkopfes 3 durch ein Blutgefäß 12 in das Herz wird der Pumpenkopf 3 durch Lockerlassen der Welle oder durch Axialdruck auf die Welle radial komprimiert, d. h. in den Zustand seiner geringstmöglichen radialen Ausdehnung gebracht.

Ist der Pumpenkopf an dem gewünschten Ort angekommen, so kann das Pumpengehäuse durch Aufbringen eines Zuges in Richtung des Pfeils 13 axial zusammengezogen und dadurch radial aufgebläht werden, wie durch die Pfeile 14, 15 angedeutet.

Denkbar ist auch das Komprimieren und Aufblähen des Gehäuses durch Verformung des Gehäuses mittels Nutzung von Formgedächtniswerkstoffen. Hierbei wird das Sprungverhalten von Formgedächtniswerkstoffen bei bestimmten Temperaturen ausgenutzt. Durch die Schlitze 16, 17, die in axialer Richtung der Welle 6 verlaufen, kann durch das Pumpengehäuse 5 hindurch Fluid, also im vorliegenden Fall Blut, zu dem Rotor 18 der Pumpe gelangen und durch diesen weiter befördert werden, beispielsweise axial durch die Kanüle 4. In Fig. 2 ist die Anströmung des Rotors radial ausgeführt. In Fig. 2a ist schematisch eine Ausführung mit axialer An- und Abströmung dargestellt.

Der Rotor weist einen Rotorblattträger 19 sowie Rotorblätter 20, 21 auf, wobei die Rotorblätter 20, 21 im Pumpbetrieb, d.h. im expandierten Zustand des Rotors aufgeklappt sind.

Der Radius des Rotors im Betrieb ist auf den Innendurchmesser des Pumpgehäuses in dessen aufgeblähtem Zustand abgestimmt.

Soll der Pumpenkopf aus dem Herz 1 entfernt werden, so wird der Pumpbetrieb eingestellt und die Rotorblätter 20, 21 legen sich an den Rotorblattträger 19 zur Verringerung des Radius des Rotors 18 an. Dies wird vorteilhaft durch eine Rotation des Rotors 18 in der dem Pumpbetrieb entgegengesetzten Drehrichtung unterstützt.

Wird dann die Welle 16 zum Pumpenkopf 3 nach Art eines Bowdenzuges verschoben, so nimmt der Pumpenkopf wieder seine komprimierte Form ein und kann durch das Blutgefäß 12 entfernt werden.

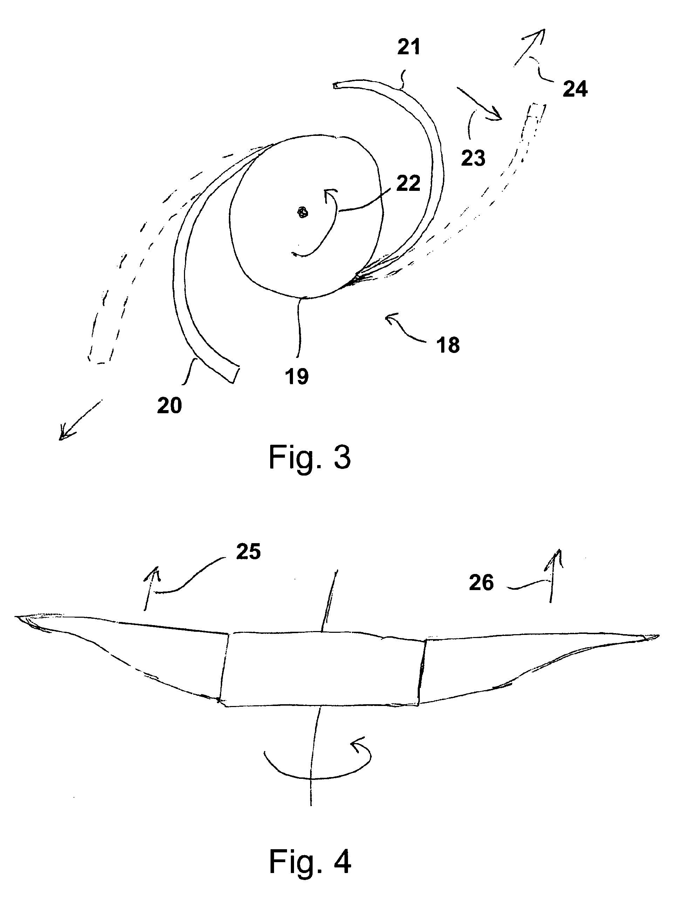

Die Fig. 3 zeigt im Detail eine Draufsicht auf den Rotor 18 mit dem Rotorblattträger 19 und den Rotorblättern 20, 21, wobei diese in durchgezogener Form in ihrem ersten Zustand, d.h. dem komprimierten Zustand des Rotors, dargestellt sind. Die Rotorblätter können in dem ersten Zustand auch noch enger an dem Rotorblattträger 19 anliegen.

Wichtig ist, dass bei Aufnahme des Pumpbetriebes und Drehung des Rotors 18 in der zum Förderbetrieb notwendigen Drehrichtung 22 ein Fluidgegendruck in Richtung des Pfeils 23 gegen die Rotorblätter entsteht und diese unter Aufweitung des Radius des Rotors 18 aufgebogen werden. Ist die Pumpe als Radialpumpe ausgelegt, so wird das Fluid radial nach außen in Richtung des Pfeils 24 verdrängt und damit gefördert.

Sind die Rotorblätter 20, 21 in Axialrichtung profiliert, so kann das Fluid auch in der Axialrichtung gefördert werden, wie in Fig. 4 durch die Pfeile 25, 26 angedeutet.

Wird der Rotor in einer der zur Förderung notwendigen Drehrichtung 22 entgegengesetzten Drehrichtung betrieben, so entsteht auf die Rotorblätter 20, 21 ein Fluidgegendruck, der der Richtung 23 entgegengesetzt ist und der zum Anklappen der Rotorblätter an den Rotorblattträger 19 und zu einer entsprechenden Reduzierung des Rotordurchmessers führt. In diesem Zustand kann der Rotor mit entsprechend komprimiertem Pumpengehäuse 5 aus dem Herzen durch die Blutbahn entfernt werden.

Durch die Wahl der Drehrichtung und der Drehzahl kann somit einerseits der Durchmesser des Rotors gezielt verändert werden, andererseits die Förderleistung der Pumpe wunschgemäß eingestellt werden.

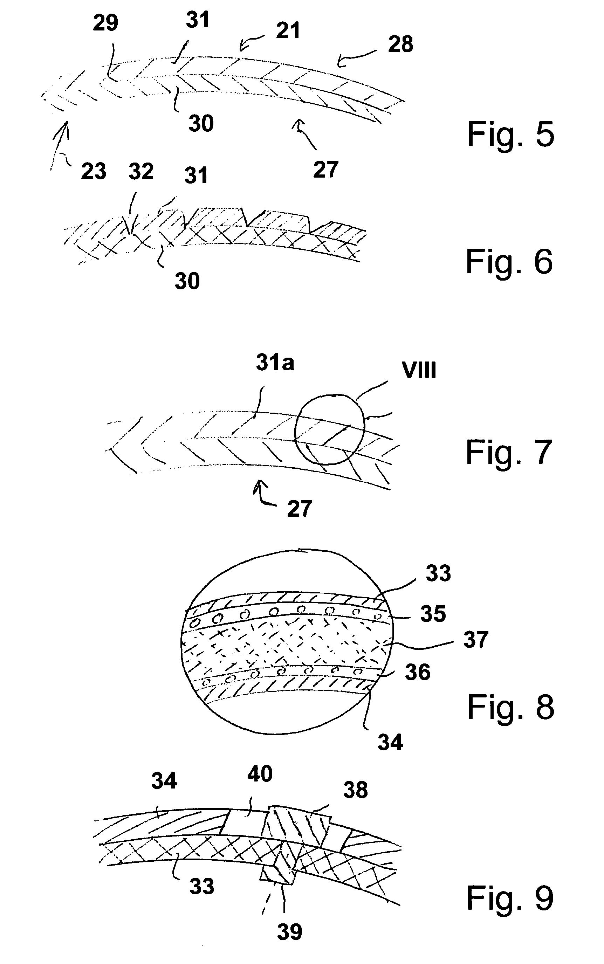

Die Fig. 5 zeigt beispielhaft ein Rotorblatt 21 mit einer im Pumpenbetrieb vorlaufenden Seite 27 sowie einer nachlaufenden Seite 28, wobei das Rotorblatt entlang einer Trennfläche 29 auf deren beiden Seiten unterschiedliche Beschaffenheiten aufweist. Im Betrieb wirkt auf das Rotorblatt ein Fluidgegendruck in Richtung des Pfeils 23 und verformt dieses in den zweiten Zustand in dem der Rotor expandiert ist. Hierzu muss die vorlaufende Seite 27 in einem gewissen Maß dehnbar sein und die entsprechende erste Materialschicht 30 weist aus diesem Grunde Membraneigenschaften auf. Es kann sich bei dieser ersten Materialschicht beispielsweise um Gummi oder ein elastisches Kunststoffmaterial handeln, das bis zu einer Dehngrenze elastisch verformbar ist und sich möglichst danach einer weiteren Dehnung widersetzt.

Auf der nachlaufenden Seite 21 besteht die zweite Materialschicht 31 aus einem druckfesten Material, das beispielsweise so hart gestaltet ist, dass es sich bei den im Betrieb wirkenden Kräften nur minimal verformt, so dass die Biegung des Rotorblattes ausschließlich über die Dehnung der ersten Materialschicht 30 realisiert wird.

Es kann jedoch eine gewisse Kompressibilität der zweiten Materialschicht 31 vorgesehen sein.

Die Fig. 6 zeigt ein weiteres Beispiel zur Gestaltung eines Rotorblattes, bei dem in der zweiten Materialschicht 31 Einkerbungen 32 vorgesehen sind, die die Kompression und Biegung der nachlaufenden Seite erlauben, bis dass die Kerben 32 geschlossen sind und die verschiedenen zwischen den Kerben 32 gebildeten Stege formschlüssig aneinanderstoßen. In diesem Zustand würde eine weitere Biegung des Rotorblattes gestoppt.

Das Material der ersten Materialschicht 31 kann in diesem Fall ebenfalls ein harter Kunststoff sein, aus dem Teile ausgeschnitten oder in einen Guss- oder Prägevorgang ausgespart werden.

Auch in diesem Fall besteht das Material der ersten Materialschicht 30 aus einem begrenzt dehnbaren Stoff.

In der Fig. 7 ist ein Rotorblatt im Querschnitt dargestellt, wobei die Einzelheit VIII in der Fig. 8 detaillierter gezeigt ist. Die Einzelheit VIII zeigt dabei die druckfeste zweite Materialschicht 31a, die ihrerseits mehrschichtig aufgebaut ist nach Art einer Sandwichstruktur, wobei diese aus zug- und/oder druckfesten Außenschichten 33, 34 35, 36 sowie einer Volumenschicht 37 besteht. Die Außenschichten 35, 36 können beispielsweise gewebeverstärkt sein.

Damit wird auf der nachlaufenden Seite eine sehr druckfeste Schicht gebildet, so dass die Verformbarkeit des Rotorblattes im Wesentlichen durch die Dehnbarkeit der vorlaufenden Seite 27 bestimmt ist.

In der Fig. 9 ist eine Variante dargestellt, bei der ein Anschlagselement 38 in der ersten Schicht 30 befestigt ist, beispielsweise mittels einer eingelassenen Schraube 39, wobei das Anschlagselement 38 in eine Öffnung 40 der zweiten Schicht 31 hineinragt.

Wird das Rotorblatt 21 verformt, so wird sich die Öffnung 40 in der zweiten Materialschicht 31 tendenziell verkleinern und verschieben, bis die Ränder der Öffnung 40 an das Anschlagselement 38 anschlagen. Das Anschlagselement besteht aus einem harten Material, ebenso wie die zweite Materialschicht 31, so dass nach dem Anschlagen keine weitere Kompression auf der nachlaufenden Seite möglich ist und das Schaufelblatt sich gegen weitere Verformung versteift.

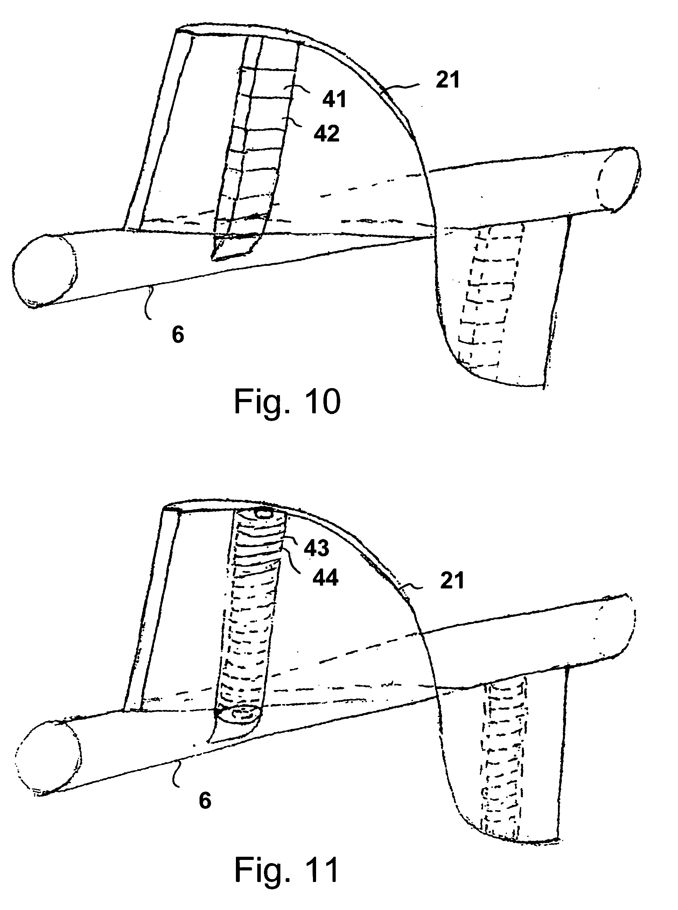

Fig. 10 zeigt ein helixförmiges Rotorblatt, bei dem eine Reihe von Formelementen 41, 42 auf der nachlaufenden Seite des Blattes mit diesem verbunden, beispielsweise aufgeklebt oder mit anderen Fügeverfahren aufgebracht ist. Im komprimierten Zustand des Rotors ist zwischen den Formelementen jeweils Abstand. Beim Betrieb der Pumpe und nach Aufrichten des Blattes liegen die Formelemente aneinander an und versteifen sich als durchgehender Steg, der die als Membran wirkenden Flächenteile des Blattes stützt und eine weitere Verformung verhindert. Mehrere solche Reihen von Formelementen können entlang der Antriebswelle 6 axial und azimutal versetzt angeordnet sein.

Eine ähnliche Konstruktion ist in Fig. 11 gezeigt, wo der Steg zur Verfestigung des Rotorblattes durch eine aus Windungen bestehende Wicklung, beispielsweise bestehend aus einem Kunststoff, einem Federdraht oder aus einem Schlauch gebildet ist. Die einzelnen Windungen bilden je ein Formelement und sind einzeln mit der membranartigen Fläche des Rotorblattes durch Kleben verbunden. Beim Komprimieren des Rotors öffnen sich die Zwickel zwischen den Windungen, und diese schließen sich beim Aufrichten des Blattes. Um die Wicklung zu stabilisieren, ist innerhalb dieser ein durchgehender Kern vorgesehen, der flexibel sein kann.

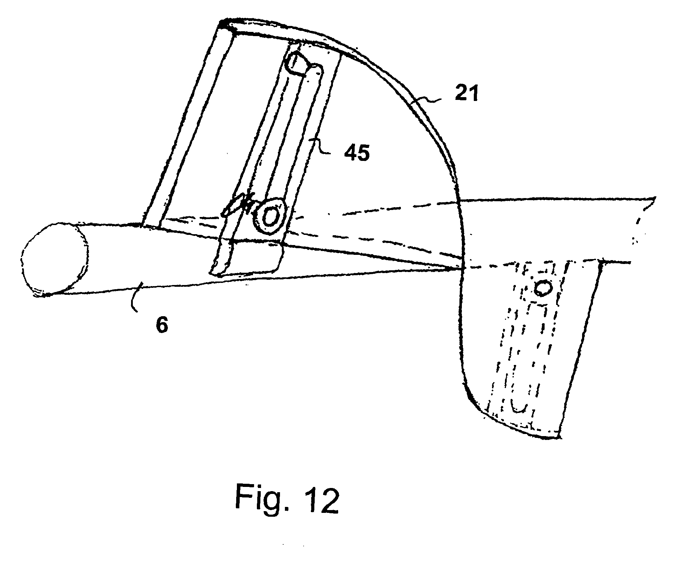

Die Fig. 12 zeigt die Stützung eines Rotorblattes durch eine feste Schiene/Kulisse 45, in der ein Anschlagselement begrenzt beweglich ist. Das Anschlagselement ist mit dem Rotorblatt verbunden.

Die Schiene/Kulisse 45 kann bezogen auf die zu erwartenden angreifenden Kräfte und Momente als knickfestes und druckfestes Bauteil ausgeführt sein. Durch die Biegung entstehen bei dieser Ausführung geringe zusätzliche Rückstellkräfte. Durch die geringe Materialstärke werden absolut gesehen wenig Rückstellkräfte erzeugt.

In Fig. 12 befindet sich das Anschlagselement in der unteren Position. Ein Verbiegen bis zum Knickfall würde für diese Position durch die geringe Länge zwischen Kulissenaufnahme an der Welle 6 und Position des Führungsstiftes in der Schiene/Kulisse 45 hohe angreifende Kräfte erfordern.

Die genannten und beschriebenen Konstruktionen von Rotorblättern sind Beispiele dafür, wie durch unterschiedliche Gestaltung der verschiedenen Seiten der Rotorblätter eine begrenzte Verformbarkeit durch den Fluidgegendruck im Betrieb erreicht werden kann.

Bei einer Rotation des Rotors in einer der Betriebsrichtung entgegengesetzten Richtung wird die Verformung der Rotorblätter rückgängig gemacht, und diese legen sich an den Rotor an, nehmen einen ersten Zustand an und definieren damit den komprimierten Zustand des Rotors, in dem dieser durch eine enge Öffnung, beispielsweise ein Blutgefäß oder einen röhrenförmigen künstlichen Zugang (Schleuse)), komfortabel bewegt werden kann.

Damit erlaubt die Erfindung in konstruktiv besonders einfacher Weise die Realisierung eines in seinem Durchmesser veränderbaren Rotors für verschiedene Anwendungen, besonders vorteilhaft jedoch für den medizinischen Bereich.

## Patentansprüche

1. Fluidpumpe, insbesondere Flüssigkeitspumpe mit einem Rotor (18) mit wenigstens einem Rotorblatt (20, 21) zur Förderung des Fluids, wobei der Rotor bezüglich seines Durchmessers zwischen einem ersten, komprimierten Zustand, und einem zweiten, expandierten Zustand veränderbar ist,
**dadurch gekennzeichnet,**
**dass** das wenigstens eine Rotorblatt (20, 21) zwischen einem ersten Zustand, den es im komprimierten Zustand des Rotors einnimmt, und einem zweiten Zustand, den es im expandierten Zustand des Rotors einnimmt, durch einen Fluidgegendruck (23) bei einer Rotation des Rotors im Pumpbetrieb verformbar ist.

2. Fluidpumpe nach Anspruch 1, **dadurch gekennzeichnet, dass** das wenigstens eine Rotorblatt (20, 21) durch den Fluidgegendruck (23) elastisch verformbar ist.

3. Fluidpumpe nach Anspruch 1 oder 2, **dadurch gekennzeichnet, dass** die Verformbarkeit des wenigstens einen Rotorblatts (20, 21) durch Materialeigenschaften und/oder den konstruktiven Aufbau des Rotorblattes begrenzt ist.

4. Fluidpumpe nach Anspruch 3, **dadurch gekennzeichnet, dass** die Verformbarkeit des wenigstens einen Rotorblatts (20, 21) derart begrenzt ist, dass die Verformung nicht über die Form hinausgeht, bei der der Rotor den größtmöglichen Fluidgegendruck erzeugt.

5. Fluidpumpe nach Anspruch 1 oder einem der folgenden, **dadurch gekennzeichnet, dass** das wenigstens eine Rotorblatt (20, 21) eine bei Förderbetrieb in Bewegungsrichtung vorlaufende (27) und eine nachlaufende Seite (28) aufweist, die verschieden gestaltet sind.

6. Fluidpumpe nach Anspruch 5, **dadurch gekennzeichnet, dass** die vorlaufende Seite (27) des wenigstens einen Rotorblatts (20, 21) aus einem ersten Material und die nachlaufende Seite (28) aus einem zweiten, von dem ersten verschiedenen Material besteht.

7. Fluidpumpe nach Anspruch 5 oder 6, **dadurch gekennzeichnet, dass** das erste Material dehnbarer ist als das zweite Material.

8. Fluidpumpe nach Anspruch 7, **dadurch gekennzeichnet, dass** das erste Material eine Dehngrenze aufweist.

9. Fluidpumpe nach Anspruch 7 oder 8, **dadurch gekennzeichnet, dass** in das erste Material Fasern eingebettet sind, die wesentlich dehnfester sind als das erste Material.

10. Fluidpumpe nach Anspruch 5 oder einem der folgenden, **dadurch gekennzeichnet, dass** das zweite Material inkompressibel ist.

11. Fluidpumpe nach einem der Ansprüche 5 bis 9, **dadurch gekennzeichnet, dass** das zweite Material elastisch bis zu einer Kompressionsgrenze komprimierbar ist.

12. Fluidpumpe nach Anspruch 5 oder einem der folgenden, **dadurch gekennzeichnet, dass** das wenigstens eine Rotorblatt (20, 21) auf der nachlaufenden Seite (28) Formelemente (31, 41, 42, 43, 44) aufweist oder mit Formelementen verbunden ist, die im ersten Zustand voneinander beabstandet sind und die im zweiten Zustand aneinander anliegen.

13. Fluidpumpe nach Anspruch 12, **dadurch gekennzeichnet, dass** die Formelemente (31, 41, 42, 43, 44) im ersten Zustand durch Schlitze (32) voneinander getrennt sind.

14. Fluidpumpe nach Anspruch 12 oder 13, **dadurch gekennzeichnet, dass** die Formelemente (41, 42) auf eine Seite, insbesondere die nachlaufende Seite eines Rotorblattes aufgeklebt oder mit einem anderen Fügeverfahren aufgebracht sind.

15. Fluidpumpe nach Anspruch 12, 13 oder 14, **dadurch gekennzeichnet, dass** die Formelemente (43, 44) durch je eine Windung einer Wicklung gebildet sind.

16. Fluidpumpe nach Anspruch 12 oder einem der folgenden, **dadurch gekennzeichnet, dass** die Formelemente (31, 41, 42, 43, 44) in ein komprimierbares Material eingebettet sind.

17. Fluidpumpe nach Anspruch 5 oder einem der folgenden, **dadurch gekennzeichnet, dass** wenigstens ein Anschlagselement (38, 39) auf einer Seite des wenigstens einen Rotorblatts befestigt ist, welches die Trennfläche (29) zwischen der vorlaufenden Seite und der nachlaufenden Seite durchsetzt und auf der anderen Seite des Rotorblattes in einer Ausnehmung begrenzt beweglich ist.

18. Verfahren zum Betrieb einer Fluidpumpe nach einem der Ansprüche 1 bis 17, **dadurch gekennzeichnet, dass** die Pumpe durch Rotation des Rotors (18) in Betriebsrichtung gestartet und der Rotor durch den Fluidgegendruck (23) expandiert wird.

19. Verfahren zum Betrieb einer Fluidpumpe nach einem der Ansprüche 1 bis 15, **dadurch gekennzeichnet, dass** zur Verringerung des Rotordurchmessers dieser in der der Betriebsrichtung (22) entgegengesetzten Richtung angetrieben wird.

20. Verfahren zum Betrieb einer Fluidpumpe nach einem der Ansprüche 1 bis 15, **dadurch gekennzeichnet, dass** der Rotor (18) während des Durchführens der Pumpe durch eine Öffnung, insbesondere ein Blutgefäß oder ein röhrenförmiger künstlicher Zugang, in der der Betriebsrichtung entgegengesetzten Richtung (22) angetrieben wird.
